(19) Europäisches Patentamt / European Patent Office / Office européen des brevets

(11) **EP 4 620 710 A1**

(12) **EUROPEAN PATENT APPLICATION**
published in accordance with Art. 153(4) EPC

(43) Date of publication:
24.09.2025 Bulletin 2025/39

(21) Application number: 23890569.9

(22) Date of filing: 31.10.2023

(51) International Patent Classification (IPC):
*B60H 3/00* (2006.01)   *A61L 9/20* (2006.01)
*B01D 53/76* (2006.01)   *A61L 9/015* (2006.01)

(52) Cooperative Patent Classification (CPC):
A61L 9/015; A61L 9/12; A61L 9/20; B01D 53/72;
B01D 53/76; B60H 3/00; Y02A 50/20

(86) International application number:
PCT/CN2023/128149

(87) International publication number:
WO 2024/104133 (23.05.2024 Gazette 2024/21)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 17.11.2022 CN 202211439112

(71) Applicant: Guangdong Arcair Appliance Co., Ltd.
Guangdong 528318 (CN)

(72) Inventors:
• KANG, Zuotian
  Foshan, Guangdong 528318 (CN)
• TANG, Haijiang
  Foshan, Guangdong 528318 (CN)
• LIANG, Haisheng
  Foshan, Guangdong 528318 (CN)

(74) Representative: Ipey
Apex House
Thomas Street
Trethomas
Caerphilly CF83 8DP (GB)

(54) **GAS PURIFICATION METHOD AND GAS PURIFIER**

(57) The present disclosure provides a gas purification method and a gas purifier. The gas purification method includes: sucking external gas into an inner housing; supplying ozone to an interior of the inner housing to react with the external gas; irradiating the interior of the inner housing with ultraviolet rays to disinfect the external gas and decompose the ozone; discharging the purified gas out of the inner housing. After the external gas is sucked into the inner housing, ozone is supplied to the interior of the inner housing to react with the gas so as to perform sterilization and deodorization treatment; the interior of the inner housing is irradiated with the ultraviolet rays to further carry out sterilization treatment on the gas and decompose excess ozone.

Suck external gas into the inner housing

↓

Supply ozone to the interior of the inner housing to react with the external gas

↓

Irradiate the interior of the inner housing with ultraviolet rays to disinfect the external gas and decompose the ozone

↓

Discharge the purified gas out of the inner housing

FIG. 5

## Description

### TECHNICAL FIELD

The present disclosure relates to the technical field of household appliances and in particular to a gas purifier and a gas purification method.

### BACKGROUND

The existing ozone purifiers can generate ozone which reacts with the impurities in the air to achieve the sterilization and deodorizing effects. However, the remaining ozone can also cause pollution to the environment. Furthermore, the sterilization effect under single ozone reaction is not satisfying.

### SUMMARY

The present disclosure aims to, at least to some extent, solve one of the problems in the prior arts. For this reason, the present disclosure provides a gas purifier and a gas purification method.

According to a first aspect, one embodiment of the present disclosure provides a gas purification method, which includes the following steps:

at step S100, sucking external gas into the inner housing;

at step S200, supplying ozone to the interior of the inner housing to react with the external gas;

at step S300, irradiating the interior of the inner housing with the ultraviolet rays to disinfect the external gas and decompose the ozone;

at step S400, discharging the purified gas out of the inner housing.

The gas purification method of the embodiments of the present disclosure at least has the following technical effects: after the external gas is sucked into the inner housing, ozone is supplied to the interior of the inner housing to react with the gas so as to carry out sterilization and deodorization treatment; the interior of the inner housing is irradiated with ultraviolet rays to further perform sterilization treatment on the gas and decompose excess ozone.

In some embodiments of the present disclosure, supplying ozone to the interior of the inner housing to react with the external gas includes:

detecting the flow rate of the gas in the inner housing; when the flow rate of the gas in the inner housing is greater than a first preset value, supplying ozone to the interior of the inner housing to react with the external gas.

In some embodiments of the present disclosure, supplying ozone to the interior of the inner housing to react with the external gas includes:

detecting the flow rate of the gas in the inner housing; when the flow rate of the gas in the inner housing is less than a second preset value, stopping supplying ozone to the interior of the inner housing.

In some embodiments of the present disclosure, irradiating the interior of the inner housing with the ultraviolet rays to carry out disinfection treatment on the external gas and decompose ozone includes:

detecting the flow rate of the gas in the inner housing; when the flow rate of the gas in the inner housing is greater than a third preset value, irradiating the interior of the inner housing with the ultraviolet rays.

In some embodiments of the present disclosure, irradiating the interior of the inner housing with the ultraviolet rays to carry out disinfection treatment on the external gas and decompose ozone includes:

detecting the flow rate of the gas in the inner housing; when the flow rate of the gas in the inner housing is greater than a fourth preset value, stopping irradiating the interior of the inner housing with the ultraviolet rays.

In some embodiments of the present disclosure, the method further includes: after stopping supplying ozone to the interior of the inner housing, stopping irradiating the interior of the inner housing with the ultraviolet rays.

In some embodiments of the present disclosure, after stopping supplying ozone to the interior of the inner housing, stopping irradiating the interior of the inner housing with the ultraviolet rays includes:

after stopping supplying ozone to the interior of the inner housing, with elapse of a first predetermined time, stopping irradiating the interior of the inner housing with the ultraviolet rays.

In some embodiments of the present disclosure, after stopping supplying ozone to the interior of the inner housing, stopping irradiating the interior of the inner housing with the ultraviolet rays includes:

after stopping supplying ozone to the interior of the inner housing, detecting the flow rate of the gas in the inner housing; when the flow rate of the gas in the inner housing is less than a fifth preset value, stopping irradiating the interior of the inner housing with the ultraviolet rays.

According to a second aspect, one embodiment of the present disclosure further provides a gas purifier, which includes:

an inner housing, provided with an air inlet and an air outlet;

an ozone generator, mounted inside the inner housing and located between the air inlet and the air outlet to supply ozone;

a disinfection lamp, mounted inside the inner housing and located between the ozone generator and the air outlet to emit ultraviolet rays for disinfection and ozone decomposition;

a gas flow sensor, disposed inside the inner housing and electrically connected with a control assembly.

The gas purifier of the embodiments of the present disclosure at least has the following technical effects: after the external gas enters the inner housing via the air inlet, the gas reacts with the ozone generated by the ozone generator to go through sterilization and deodorization treatment, and next, the ultraviolet rays emitted by the disinfection lamp further perform sterilization treatment on the gas and decompose excess ozone. The purified gas is then discharged through the air outlet.

In some embodiments of the present disclosure, the gas purifier further includes a control assembly which is electrically connected with the ozone generator and the disinfection lamp respectively.

The additional aspects and advantages of the present disclosure will be partly given in the following descriptions and will partly become obvious from the following descriptions or be understood from the practice of the present disclosure.

## BRIEF DESCRIPTION OF THE DRAWINGS

The above and/or additional aspects and advantages of the present disclosure will become obvious and easy to understand from the descriptions of the embodiments made in combination with the drawings.

FIG. 1 is a structural schematic diagram illustrating a gas purifier according to some embodiments of the present disclosure.

FIG. 2 is an exploded view illustrating a gas purifier according to some embodiments of the present disclosure.

FIG. 3 is a structural schematic diagram illustrating a gas purifier with an outer housing according to some embodiments of the present disclosure.

FIG. 4 is a schematic diagram illustrating the interior of a gas purifier according to some embodiments of the present disclosure.

FIG. 5 is a flowchart illustrating a gas purification method according to some embodiments of the present disclosure.

FIG. 6 is a schematic diagram illustrating a molecular structure under the irradiation of ultraviolet rays.

FIG. 7 is a relationship diagram of wavelength and photon energy.

The numerals of the drawings are described below:

inner housing 100, air inlet 101, air outlet 102, upper housing 110, middle housing 120, lower housing 130, first region 141, second region 142, light channel 150, front housing 161, and rear housing 162;

outer housing 200, inlet air grating 201, outlet air grating 202;

ozone generator 310, disinfection lamp 320, and control assembly 330.

## DETAILED DESCRIPTION OF THE EMBODIMENTS

The embodiments of the present disclosure are detailed below with the examples of the embodiments illustrated in the drawings. The same or similar numerals represent same or similar elements or the elements with same or similar function throughout. The embodiments described below by referring to the drawings are illustrative and used only to interpret the present disclosure and shall not be understood as limitation of the present disclosure.

In the descriptions of the present disclosure, it should be understood that for the orientation descriptions, for example, the orientation or positional relationship indicated by upper, lower, front, back, left, right and the like is based on the orientation or positional relationship shown in the drawings, and used only for ease of descriptions of the present disclosure and simplified descriptions rather than for indicating or implying the indicated device or element must have specific orientation or be constructed or operated in a specific orientation, and thus shall not be understood as limitation of the present disclosure.

In the descriptions of the present disclosure, the plural refers to two or more, the greater than, less than and more than and the like are understood as excluding the present number, and the above, below and within and the like are understood as including the present number. The descriptions of the first and second are used only to distinguish technical features and shall not be understood as indicating or implying relevant importance or implicitly indicating the number of the indicated technical features or implicitly indicating the precedence of the indicated technical features.

In the descriptions of the present disclosure, unless otherwise clearly stated, the terms such as dispose, mount and connect and the like shall be understood in broad sense, and those skilled in the arts can reasonably determine the specific meanings of the above terms in the present disclosure based on the specific contents of the technical solutions.

The embodiments of the present disclosure will be further elaborated below in combination with the accompanying drawings.

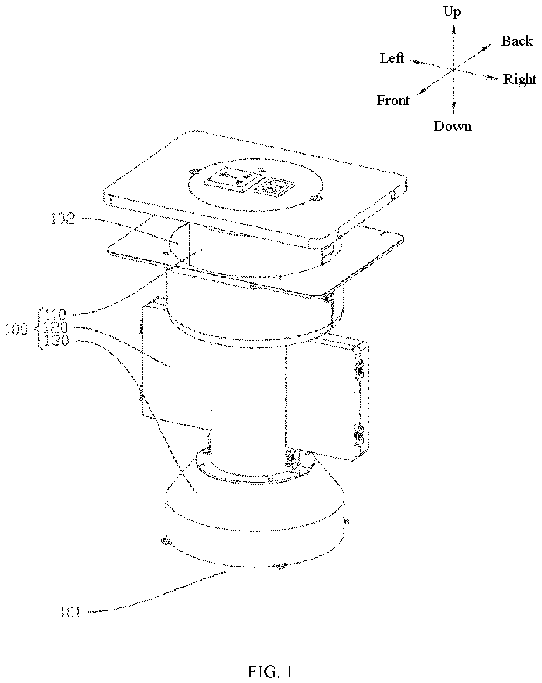

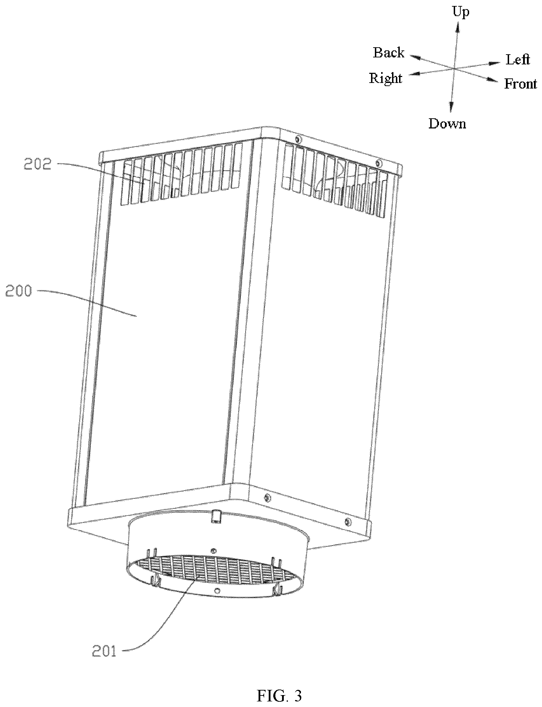

In some embodiments of the present disclosure, with reference to FIGS. 1 to 4, the gas purifier includes an inner housing 100, an ozone generator 310 and a disinfection lamp 320. The inner housing 100 is provided with an air inlet 101 and an air outlet 102. The ozone generator 310 is mounted inside the inner housing 100 and located between the air inlet 101 and the air outlet 102 to provide ozone. The disinfection lamp 320 is mounted inside the inner housing 100 and located between the ozone generator 310 and the air outlet 102 to emit ultraviolet rays for disinfection and ozone decomposition.

When the external gas enters the inner housing 100 via the air inlet 101, the gas reacts with the ozone generated by the ozone generator 310 to go through disinfection and deodorization treatment. Next, the ultraviolet rays emitted by the disinfection lamp 320 can further perform sterilization treatment on the gas and decompose the excess ozone, and the purified gas is discharged via the air outlet 102.

The ozone can oxidize and decompose an enzyme necessary for glucose decomposition in the bacteria, which disables TriCarboxylic Acid (TCA) cycle, and thus disables the supply of Adenosine Triphosphate (ATP) necessary for the life activities of the cells, leading to the bacteria inactivation and death.

The ozone is a high-efficiency broad-spectrum bactericide which has an extremely strong sterilization on bacteria, viruses, molds, endospores, fungi and microorganisms and the like. The ozone has a bactericidal ability 20% higher than the ultraviolet rays, and can spread in the air to realize comprehensive sterilization, with its sterilization rate 600 times faster in the water than chlorine. Its virus inactivation mechanism is carried out by directly destroying its (Ribonucleic Acid) RNA or DeoxyriboNucleic Acid (DNA) substance. But, for the sterilization of the bacteria and mold microorganisms, the ozone firstly acts on cell nucleus and then destroys the in-membrane tissues to its death.

The ozone has an oxidizing ability twice that of chlorine and the sterilization rate 600-3000 times faster than chlorine, and can even kill the bacteria within seconds. Further, the ozone is a green and environment-friendly element, which can be self reduced to oxygen and water in sterilization and disinfection process, without leaving any residues and secondary pollution.

The ozone can kill the vegetative forms of bacteria, diseased endospores and fungi, destroy the botulinum fungi and also can clear up and kill toxins and bacteria in the air, water and foods so as to remove odors, for example, can kill those common bacteria such as escherichia coli, streptococcus, pseudomonas aeruginosa, staphylococcus aureus, and toxic bacteria and the like, with its sterilization rate reaching above 99% in 15 minutes in the ozone environment.

The main composition of the waste pollution gas includes ammonia, hydrogen sulfide, thiol, sulfide, phenol, amine, amide, indole, alkane, alkene, alkyne, aromatic hydrocarbon, alcohol, aldehyde, ketone and volatile organic compound (VOC) of organic acid and inorganic substance, where hydrogen sulfide ($H_2S$ is the main source of the odor) contains the largest amount of ammonia. The ozone generates non-toxic and odorless substances by destroying the molecular structures of the odorous substances. The staged oxygen (nascent oxygen) generated by the ozone decomposition has a strong oxidizing property, and therefore, in the decomposition process, the odors and toxic gases in the organic pollutants and the like can be decomposed to generate air in short time and become stable and odorless. The chemical reaction between ozone and formaldehyde is $2O_3+HCHO \rightarrow 2O_2+H_2O_2+CO_2$, and the chemical reaction between ozone and benzene is $C_6H_6+5O_3\text{-}6CO_2+3H_2O$.

The sterilization effects of the ozone reacting with several types of organisms are shown below.

Table 1

| Category | Name | Test conditions | Ozone concentration (ppm) | Acting time | Killing rate |
|---|---|---|---|---|---|
| Bacteria | escherichia coli | in the 25°C air | 0.05 | 30 minutes | 100% |
| | pseudomonas aeruginosa | | 0.08-0.6 | 30 minutes | 99.9% |
| | staphylococcus aureus | in the air | 9 | 60 minutes | 99.97% |
| | staphylococcus albus | | 10 | 3 minutes | 99.99% |
| | pseudomonas fluorescens | phosphate buffer solution | ozone introduction | 15 seconds | 100% |
| | Salmonella typhimurium | | | | |
| | F's dysentery bacillus | | | | |
| | Vibrio cholerae | | | | |
| | escherichia coli | 10°C | 0.3 | 1 minute | 100% |
| Endospore | bacillus subtilis variant | in the air | 2.5mg/L | 1 minute | 100% |
| | bacillus subtilis variant | in water | 4.6mg/L | 3 minutes | 100% |
| Viruses | HBsAg | in water | 13.6 | 30 minutes | 99.99% |
| | HAsAg | | | | 100% |
| | HBsAg | in the air | 0.3 | 30 minutes | 75% |
| | Influenza A virus | | 0.5 | | 100% |
| | poliovirus | in water | 0.13 | 3 minutes | 100% |
| | escherichia coli phage | | 0.09-0.8 | 3 minutes | drop by 7 log values |
| | simian rotavirus | | 0.25 | | 100% |
| | human rotavirus | | | | |
| | human immunodeficiency virus (HIV) | in serum | 4 | drop by 6 log values in titer | |
| Fungi | aspergillus versicolor | in the air | 9.6 | 100 minutes | 100% |
| | cladosporium herbarum | | 23 | 30 minutes | |
| | penicillium nigricans | | 12.5 | 35 minutes | |
| | penicillium citrinum | | 15.4 | 30 minutes | |
| | fusarium oxysporum | | 15.5 | 20 minutes | |
| | penicillium | in the air | in the air | 30 minutes | 93.8% |
| | mucor | in water | | | |
| | aspergillus niger | | 1.5 | 1 minute | 100% |

The ultraviolet rays are divided into four wave bands based on wavelength: long-wave UVA, medium-wave UVB, short-wave UVC, and vacuum-wave UVD.

The long-wave UVA with a wavelength of 320 to 400nm has strong ability to penetrate through glass and even the 9-foot water. It is always present throughout the year whether sunny or cloudy, or whether at dusk or dawn. The degree of the harm to the human body: more than 95% of the ultraviolet rays exposed to the skin in daily life are UVA which therefore can bring the largest harm to the skin. The UVA can penetrate through the epidermis to attack the dermis, severely harming the bone collagen and elastin in the skin. Furthermore, the dermal cells have poor self protection ability and even a small amount of UVA can bring a severe harm to them. After a long time, the skin may suffer from the problems such as sagging, wrinkling and micro-vessel floating and the like. Also, it can activate tyrosinase, which leads to instant melanin deposition and new melamin formation, making the skin dark and lackluster. The UVA can bring long-term, chronic and lasting harm so that the skin is aged early. The UVA is also called aging rays.

The UVA application field: the UVA ultraviolet rays of 360nm wavelength are consistent with the phototactic reaction curve of the insects and thus the insect trap lamps can be made based on the characteristics. The UVA ultraviolet rays of the wavelength of 300 to 420nm can penetrate through specially-tinted glass lamp tubes with the visible light fully cut off, and only radiate near ultraviolet light with 365nm as center for use in the places such as ore identification, stage decoration and fake currency detection and the like.

The medium-wave UVB with a wavelength of 275 to 320nm has a medium penetration ability. Its part with short wavelength can be absorbed by transparent glass. The medium-wave ultraviolet rays in the sun light are mostly absorbed by the ozone layer and only less than 2% can reach the surface of the earth, with the strong intensity in summer and after noon. The degree of the harm to the human body: it can oxidize the protective lipid layer of the epidermis and dry the skin. Further, the nucleic acid and proteins in the epidermal cells are denatured, bringing symptoms such as acute dermatitis (i.e. sunburn) and the like. The skin will become red and painful. Worse, for example, the long time exposure to sun shine may lead to skin canceration. Furthermore, the long time harm of the UVB can also cause melanin cells to mutate, leading to sun spots difficult to remove.

The UVB application field: the ultraviolet health care lamps and the plant growth lamps are made of the special ultraviolet-transmitting glass (the light below 254nm is not transmitted) and the fluorescent powder with the peak value around 300nm.

The short-wave UVC with a wavelength of 200 to 275nm is also called short-wave sterilization ultraviolet rays. It has the weakest penetration ability and is unable to penetrate through most transparent glass and plastic. The short-wave ultraviolet rays in the sun light are almost fully absorbed by the ozone layer before reaching the ground surface. The extent of the harm to the human body: since the UVC in the nature is absorbed by the ozone layer before reaching to the ground surface, its impact on the skin can be neglected. As a matter of fact, the short-wave ultraviolet rays have significant harm to the human body and shall not be directly irradiated onto the human body. If the short-wave ultraviolet rays are directly irradiated onto human body, the skin may be burnt in a short time of irradiation. Long-time or high-intensity irradiation can also lead to skin cancer.

The UVC application field: the ultraviolet sterilization lamps emit the UVC short-wave ultraviolet rays. The short-wave ultraviolet rays are widely applied to the fields such as hospitals, air conditioning systems, disinfection cabinets, water treatment equipment, water dispensers, waste water treatment plants, swimming pools, food and beverage processing and packaging equipment, foods factories, cosmetics factories, dairy product factories, wine brewing plants, beverage plants, bakeries, and cold storage rooms and the like.

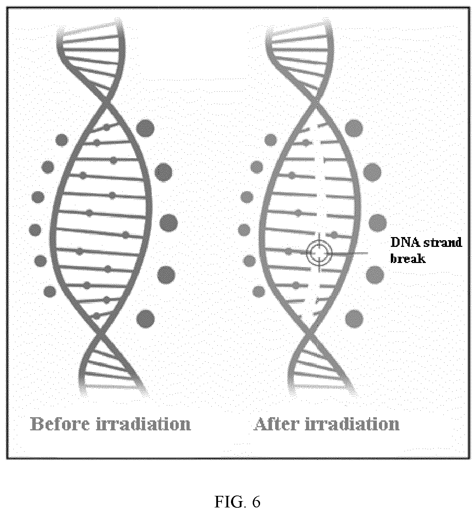

With reference to FIG. 6, the ultraviolet sterilization and disinfection is carried out by destroying the molecular structures of the DNA or RNA in the body cells of the microorganisms using the UVC ultraviolet rays to cause the vegetative cells and/or regenerative cells to die. Under a given dose, the deep ultraviolet rays can kill most bacteria and viruses within one second. Further, the ultraviolet energy can decompose ozone into oxygen.

The UVC sterilization rate is related to the target microorganism and the irradiation dose. The common vegetative forms of bacteria can be killed with the irradiation dose of $1000\mu W.s/cm^2$; the bacterial endospore can be killed with the irradiation dose of $100000\mu W.s/cm^2$; the viruses can be killed with the irradiation dose between the irradiation dose of the vegetative form of bacteria and the irradiation dose of the bacterial endospore; the irradiation dose for killing the fungal spore is higher than that for killing the bacterial endospore, and sometimes the irradiation dose needs to be $600000\mu W.s/cm^2$; the irradiation dose for killing the unknown target microorganisms should be no less than $100000\mu W.s/cm^2$. The times required for different types of organisms to be killed 100% with the irradiation dose of $30000\mu W.s/cm^2$ are shown in Table 2. For example, the bacillus anthracis can be killed 100% in only 0.3 seconds. It can be known that the ultraviolet sterilization has high efficiency.

Table 2

| Category | Name | Time for 100% killing (s) | Category | Name | Time for 100% killing (s) |
|---|---|---|---|---|---|
| Bacteria | bacillus anthracis | 0.3 | Bacteria | tubercle bacillus | 0.41 |
| | corynebacterium diphtheriae | 0.25 | | vibrio cholerae | 0.64 |
| | clostridium tetani | 0.33 | | pseudomonas | 0.37 |
| | clostridium botulinum | 0.8 | | salmonella | 0.51 |
| | dysentery bacilli | 0.15 | | enteric fever bacteria | 0.41 |
| | escherichia coli | 0.36 | | bacillus typhimurium | 0.53 |
| viruses | adenovirus | 0.1 | viruses | influenza virus | 0.23 |
| | bacteriocyte-phage virus | 0.2 | | poliovirus | 0.8 |
| | coxsackievirus | 0.08 | | rotavirus | 0.52 |
| | echovirus | 0.73 | | tobacco mosaic virus | 16 |
| | echovirus type I | 0.75 | | hepatitis B virus | 0.73 |
| Mold spores | aspergillus niger | 6.67 | Mold spores | soft spore | 0.33 |
| | aspergillus | 0.73-8.80 | | penicillium | 0.87-2.93 |
| | coprophilous fungi | 8 | | penicillium chrysogenum virus | 2-3.33 |
| | mucor | 0.23-4.67 | | other penicillium bacteria | 0.87 |
| Algae | blue-green algae | 10-40 | Algae | paramecium | 7.3 |
| | chlorella | 0.93 | | green algae | 1.22 |
| | nematode Eggs | 3.4 | | protozoa | 4-6.70 |
| Fish disease | Fung1 disease | 1.6 | Fish disease | infectious pancreatic necrosis | 4 |
| | white spot disease | 2.67 | | viral hemorrhagic disease | 1.6 |

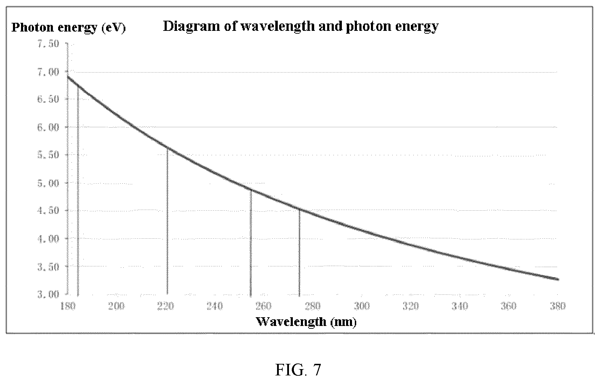

With reference to FIG. 7, it shows the relationship of wavelength and photon energy. The greater the wavelength is, the smaller the photo energy is, which satisfies $E = \frac{h}{k} \times \frac{C}{\lambda}$, where E is energy in the unit of eV (electron volt); h is Planck constant which is equal to h=$6.63 \times 10^{-34}$Js (j*s); k is a constant which is equal to k=$1.6 \times 10^{-19}$J/eV; C is light speed which is equal to $3 \times 10^{17}$nm/s, and $\lambda$ is wavelength in the unit of nm. Therefore, the ultraviolet sterilization is usually carried out by a UVC lamp tube which has short wavelength and high energy.

The bond energy of the ozone is 101.2kJ/mol, and the ozone has the largest absorption at the wavelength of 254nm and can go through dissociation reaction after absorbing the ultraviolet light: $O_3$+hv→O·+$O_2$. Under the synergic effect of the ultraviolet light, due to the formation of hydrocarbyl radicals, the ozone can effectively destroy the molecular structures of the organic substances and finally decompose them: $O_3$+$H_2O$+hv→$O_2$+$H_2O_2$, $H_2O_2$ +hv→2·OH.

It should be noted that the gas purified in the embodiment can be applied to the fields such as kitchens, bath rooms, and bed rooms and the like. After the gas purified is connected with an exhaust fan, the exhaust fan pumps gas into the gas purifier and the gas purifier performs disinfection, sterilization and deodorization on the gas and then discharge the treated gas, so as to complete gas purification.

In some embodiments of the present disclosure, with reference to FIGS. 1, 3, and 4, the gas purifier further includes an outer housing 200 inside which the inner housing 100 is located. The outer housing 200 is provided with an inlet air grating 201 and an outlet air grating 202. The inlet air grating 201 is disposed at a lower end of the outer housing 200, and the outlet air grating 202 is disposed on a sidewall of the outer housing 200. The outlet air grating 202 is composed of several elongated through holes. The inlet air grating 201 is in communication with the air inlet 101, and the outlet air grating 202 is in communication with the air outlet 102. The outer housing 200 protects the inner housing 100. The inlet air grating 201 and the outlet air grating 202 can perform ventilation and heat dissipation and to some extent, prevent external impurities entering the inner housing 100. The external gas enters the outer housing 200 via the inlet air grating 201 and then enters the inner housing 100 via the air inlet 101. The gas undergoes ozone sterilization treatment and ultraviolet treatment sequentially and then runs through the air outlet 102 and the outlet air grating 202 back to the outside.

It should be noted that the shape and size of the inlet air grating 201 and the outlet air grating 202 are not limited and can be adjusted based on actual requirements.

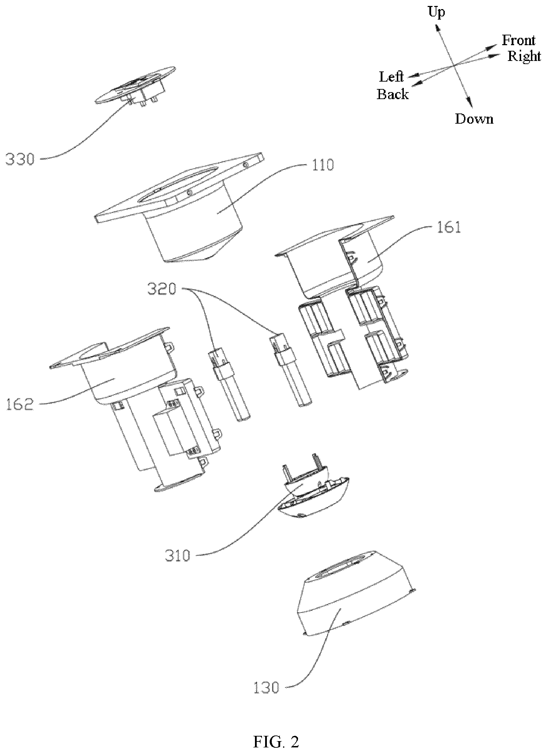

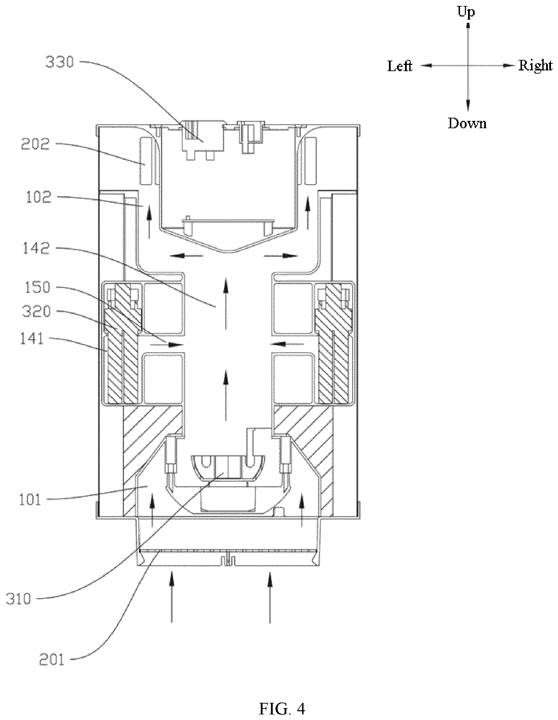

In some embodiments of the present disclosure, with reference to FIGS. 2 and 4, the gas purifier further includes a control assembly 330 which is electrically connected with the ozone generator 310 and the disinfection lamp 320 respectively. The control assembly 330 includes a power supply and a circuit board. The circuit board is electrically connected with the ozone generator 310 and the disinfection lamp 320 respectively and the power supply is used to supply power. The control assembly 330 can realize smart control on the ozone generator 310 and the disinfection lamp 320.

Preferably, the purifier further includes a gas flow sensor disposed inside the inner housing 100. The control assembly 330 is electrically connected with the gas flow sensor. The gas flow sensor measures a flow rate of the gas in the inner housing 100 and further feeds back to the control assembly 330. The control assembly 330 can control working powers of the ozone generator 310 and the disinfection lamp 320 based on the flow rate of the gas, ensuring good gas purification effect.

It can be understood that when the gas flow sensor detects the flow rate of the gas is large, the control assembly 330 increases the working power of the ozone generator 310 and the ultraviolet frequency of the disinfection lamp 320 to ensure good gas purification effect; when the gas flow sensor detects the flow rate of the gas is small, the control assembly 330 decreases the working power of the ozone generator 310 and the ultraviolet frequency of the disinfection lamp 320 so as to avoid generation of excess ozone and leakage of strong ultraviolet rays.

In some embodiments of the present disclosure, with reference to FIGS. 2 and 4, the inner housing 100 includes an upper housing 110, a middle housing 120 and a lower housing 130. The outer housing 200 is fixedly connected with the upper housing 110, the middle housing 120 and the lower housing 130 respectively. A down-facing opening of the lower housing 130 is the air inlet 101. The ozone generator 310 is disposed inside the lower housing 130. The middle housing 120 is in communication with the lower housing 130. The disinfection lamp 320 is disposed inside the middle housing 120. An opening of the middle housing 120 faces upward, and a lower end of the upper housing 110 is inserted into the opening of the middle housing 120. An annular channel between an outer circumferential wall of the upper housing 110 and an inner circumferential wall of the middle housing 120 is the air outlet 102, and the outlet air grating 202 is located above the air outlet 102.

It can be understood that, with reference to FIG. 4, after the gas in the middle housing 120 is disinfected by the ultraviolet rays emitted by the disinfection lamp 320, the gas moves upward to collide with the lower end of the upper housing 110 and then is dispersed into the air outlet 102. The annular air outlet 102 enables the gas to be fully dispersed and finally discharged to the outside through the outlet air grating 202.

In some embodiments of the present disclosure, with reference to FIG. 4, inside the middle housing 120 are a first region 141 and a second region 142. A light channel 150 is disposed between the first region 141 and the second region 142. The disinfection lamp is disposed inside the first region 141 and the second region 142 is in communication with the upper housing 110 and the lower housing 130 respectively.

Preferably, two first regions 141 are located at the left and right sides of the second region 142 respectively and the disinfection lamp 320 is disposed in each of the two first regions 141.

It should be noted that the light channel 150 is used to limit the dispersion direction of the ultraviolet rays to prevent the leakage of the ultraviolet rays and enhance the disinfection efficiency of the ultraviolet rays.

It should be further noted that the direction indicated by the arrow in FIG. 4 is a flow direction of the gas or the dispersion direction of the ultraviolet rays, where the direction indicated by the arrow on the light channel 150 is the dispersion direction of the ultraviolet rays.

In some embodiments of the present disclosure, with reference to FIG. 2, the middle housing 120 includes a front housing 161 and a rear housing 162 which can be snap-fitted together. When it is required to change the disinfection lamp 320 or clean the interior, the snap-fit connection of the front housing 161 and the rear housing 162 facilitates dismounting and separation.

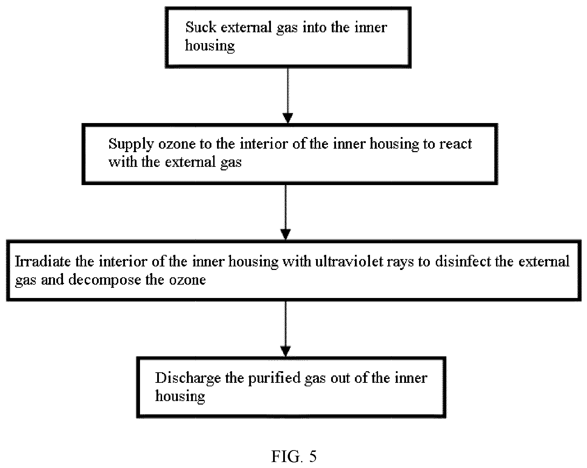

In some embodiments of the present disclosure, with reference to FIG. 5, the air purification method may include the following steps:

At step S100, external gas is sucked into the inner housing 100.

At step S200, ozone is supplied to the interior of the inner housing 100 to react with the external gas.

At step S300, the interior of the inner housing 100 is irradiated with the ultraviolet rays to disinfect the external gas and decompose the ozone.

At step S400, the purified gas in the inner housing 100 is discharged.

After the external gas is sucked into the inner housing, ozone is supplied to the interior of the inner housing to react with the gas so as to perform sterilization and deodorization treatment; the interior of the inner housing is irradiated with the ultraviolet rays to further carry out sterilization treatment on the gas and decompose excess ozone.

It can be understood that the external gas enters the inner housing 100 to firstly react with the ozone and then undergo sterilization treatment under the irradiation of the ultraviolet rays so as to finally produce the purified gas.

Preferably, supplying ozone to the interior of the inner housing 100 to react with the external gas includes:

detecting the flow rate of the gas in the inner housing 100; when the flow rate of the gas in the inner housing 100 is greater than a first preset value, supplying ozone to the interior of the inner housing 100 to react with the external gas;

detecting the flow rate of the gas in the inner housing; when the flow rate of the gas in the inner housing is less than a second preset value, stopping supplying ozone to the interior of the inner housing.

Preferably, irradiating the interior of the inner housing with the ultraviolet rays to carry out disinfection treatment on the external gas and decompose ozone includes:

detecting the flow rate of the gas in the inner housing; when the flow rate of the gas in the inner housing is greater than a third preset value, irradiating the interior of the inner housing with the ultraviolet rays;
detecting the flow rate of the gas in the inner housing; when the flow rate of the gas in the inner housing is greater than a fourth preset value, stopping irradiating the interior of the inner housing with the ultraviolet rays.

It can be understood that, when it is detected that the flow rate of the gas in the inner housing 100 is greater than the first preset value, the ozone generator 310 is started up and the ozone generated by the ozone generator reacts with the gas so as to achieve sterilization and deodorization treatment; when it is detected that the flow rate of the gas in the inner housing 100 is greater than the third preset value, the disinfection lamps 320 are turned on and the ultraviolet rays emitted by the disinfection lamps 320 perform further sterilization treatment on the gas and decompose excess ozone; when it is detected that the flow rate of the gas in the inner housing 100 is less than the second preset value, the ozone generator 310 is turned off, and at this time, the disinfection lamps 320 continue working to decompose excess ozone and sterilize the gas to prevent the external pollution of the ozone; finally, when it is detected that the flow rate of the gas in the inner housing 100 is less than the fourth preset value, the disinfection lamps 320 are turned off.

It should be noted that the first preset value may be equal to, or less than or greater than the third preset value; and the second preset value is greater than the fourth preset value.

In some embodiments of the present disclosure, the air purification method further includes: after stopping supplying ozone to the interior of the inner housing, stopping irradiating the interior of the inner housing with the ultraviolet rays.

Preferably, after stopping supplying ozone to the interior of the inner housing, stopping irradiating the interior of the inner housing with the ultraviolet rays includes:

after stopping supplying ozone to the interior of the inner housing, with elapse of a first predetermined time, stopping irradiating the interior of the inner housing with the ultraviolet rays;

after stopping supplying ozone to the interior of the inner housing, detecting the flow rate of the gas in the inner housing; when the flow rate of the gas in the inner housing is less than a fifth preset value, stopping irradiating the interior of the inner housing with the ultraviolet rays.

It can be understood that, after the ozone generator 310 is turned off, turning off the disinfection lamps 320 includes:

when the ozone generator 310 is turned off, with elapse of the first predetermined time, turning off the disinfection lamps 320; where with elapse of the first predetermined time, turning off the disinfection lamps 320 gives the disinfection lamps 320 enough time to decompose the excess ozone;

when the ozone generator 310 is turned off and the flow rate of the gas in the inner housing 100 is less than the fifth preset value, turning off the disinfection lamps 320; where the fifth preset value is a normal atmospheric flow rate, namely, the flow rate of the gas in the inner housing 100 is already consistent with the flow rate of the external gas, and turning off the disinfection lamps 320 at this time can help save energy.

In the descriptions of the present specification, the descriptions of the reference term "some embodiments" mean that the specific features, structures, materials or characteristics described in combination with the embodiments or examples are included in at least one embodiment or example of the present disclosure. In the present specification, the illustrative expressions of the above terms do not necessarily refer to same embodiments or examples. Furthermore, the specific features, structures, materials or characteristics described can be combined properly in one or more embodiments or examples.

Although the embodiments of the present disclosure have been illustrated and described, ordinary persons skilled in the arts can understand that without departing from the principle and tenet of the present disclosure, various changes, modification, substitutions and variations can be made to these embodiments, and the scope of the present disclosure shall be defined by the claims and its equivalents.

**Claims**

1. A gas purification method, comprising the following steps:

    at step S100, sucking external gas into an inner housing (100);
    at step S200, supplying ozone to an interior of the inner housing (100) to react with the external gas;
    at step S300, irradiating the interior of the inner housing (100) with ultraviolet rays to disinfect the external gas and decompose the ozone;
    at step S400, discharging the purified gas out of the inner housing (100).

2. The gas purification method of claim 1, wherein supplying ozone to the interior of the inner housing (100) to react with the external gas comprises:
    detecting a flow rate of the gas in the inner housing (100); when the flow rate of the gas in the inner housing (100) is greater than a first preset value, supplying ozone to the interior of the inner housing (100) to react with the external gas.

3. The gas purification method of claim 1, wherein supplying ozone to the interior of the inner housing (100) to react with the external gas comprises:
    detecting the flow rate of the gas in the inner housing (100); when the flow rate of the gas in the inner housing (100) is less than a second preset value, stopping supplying ozone to the interior of the inner housing (100).

4. The gas purification method of claim 1, wherein irradiating the interior of the inner housing (100) with the ultraviolet rays to carry out disinfection treatment on the external gas and decompose ozone comprises:
    detecting the flow rate of the gas in the inner housing (100); when the flow rate of the gas in the inner housing (100) is greater than a third preset value, irradiating the interior of the inner housing (100) with the ultraviolet rays.

5. The gas purification method of claim 1, wherein irradiating the interior of the inner housing (100) with the ultraviolet rays to carry out disinfection treatment on the external gas and decompose ozone comprises:
    detecting the flow rate of the gas in the inner housing (100); when the flow rate of the gas in the inner housing (100) is greater than a fourth preset value, stopping irradiating the interior of the inner housing (100) with the ultraviolet rays.

6. The gas purification method of claim 1, further comprising: after stopping supplying ozone to the interior of the inner housing (100), stopping irradiating the interior of the inner housing (100) with the ultraviolet rays.

7. The gas purification method of claim 6, wherein after stopping supplying ozone to the interior of the inner housing (100), stopping irradiating the interior of the inner housing (100) with the ultraviolet rays comprises:
    after stopping supplying ozone to the interior of the inner housing (100), with elapse of a first predetermined time, stopping irradiating the interior of the inner housing (100) with the ultraviolet rays.

8. The gas purification method of claim 6, wherein after stopping supplying ozone to the interior of the inner housing (100), stopping irradiating the interior of the inner housing (100) with the ultraviolet rays comprises:
    after stopping supplying ozone to the interior of the inner housing (100), detecting the flow rate of the gas in the inner housing (100); when the flow rate of the gas in the inner housing (100) is less than a fifth preset value, stopping irradiating the interior of the inner housing (100) with the ultraviolet rays.

9. A gas purifier, comprising:

an inner housing (100), provided with an air inlet (101) and an air outlet (102);
an ozone generator (310), mounted inside the inner housing (100) and located between the air inlet (101) and the air outlet (102) to supply ozone;
a disinfection lamp (320), mounted inside the inner housing (100) and located between the ozone generator (310) and the air outlet (102) to emit ultraviolet rays for disinfection and ozone decomposition;
a gas flow sensor, disposed inside the inner housing (100) and electrically connected with a control assembly (330).

The gas purifier of claim 1, wherein the gas purifier further comprises the control assembly (330) which is electrically connected with the ozone generator (310) and the disinfection lamp (320) respectively.

Up

Back

Left

Right

Front

Down

102

100 { 110
        120
        130

101

FIG. 1

Up

Front
Right

Left
Back

Down

330

110

161

320

162

310

130

FIG. 2

FIG. 3

FIG. 4

Suck external gas into the inner housing

↓

Supply ozone to the interior of the inner housing to react with the external gas

↓

Irradiate the interior of the inner housing with ultraviolet rays to disinfect the external gas and decompose the ozone

↓

Discharge the purified gas out of the inner housing

FIG. 5

Before irradiation    After irradiation

FIG. 6

FIG. 7

## INTERNATIONAL SEARCH REPORT

| International application No. |
| --- |
| **PCT/CN2023/128149** |

**A. CLASSIFICATION OF SUBJECT MATTER**

B60H3/00(2006.01)i; A61L9/20(2006.01)i; B01D53/76(2006.01)i; A61L9/015(2006.01)i

According to International Patent Classification (IPC) or to both national classification and IPC

**B. FIELDS SEARCHED**

Minimum documentation searched (classification system followed by classification symbols)

B60H A61L B01D

Documentation searched other than minimum documentation to the extent that such documents are included in the fields searched

Electronic data base consulted during the international search (name of data base and, where practicable, search terms used)

CNTXT, DWPI, CNKI, USTXT, EPTXT, WOTXT: 广东合捷电器股份有限公司, 康作添, 唐海江, 梁海生, 臭氧, 紫外, 传感器, 感应器, 风速, 气流, 流量, 流速, 分解, 还原, ozone, O3, UV, ultraviolet, air, gas, disinfect+ , sterilizat+

**C. DOCUMENTS CONSIDERED TO BE RELEVANT**

| Category* | Citation of document, with indication, where appropriate, of the relevant passages | Relevant to claim No. |
| --- | --- | --- |
| X | CN 111497571 A (GUANGXI ACADEMY OF SCIENCES) 07 August 2020 (2020-08-07) claims 1 and 6, description, paragraphs 7 and 53, and figures 1-2 | 1 |
| Y | CN 111497571 A (GUANGXI ACADEMY OF SCIENCES) 07 August 2020 (2020-08-07) claims 1 and 6, description, paragraphs 7 and 53, and figures 1-2 | 2-9 |
| Y | CN 113350560 A (BEIJING XINGHANG MECHANICAL-ELECTRICAL EQUIPMENT CO., LTD.) 07 September 2021 (2021-09-07) claims 1 and 6, and description, paragraph 50 | 2-9 |
| Y | CN 107158908 A (CHENGDU ZHIYA TECHNOLOGY CO., LTD.) 15 September 2017 (2017-09-15) claim 1, and figures 1-4 | 9 |
| Y | CN 110721329 A (SHENZHEN BAOWEI TECHNOLOGY CO., LTD.) 24 January 2020 (2020-01-24) claims 1-4 | 1-9 |
| Y | CN 206424767 U (SHANGHAI QUANBAO EVIRONMENTAL TECHNOLOGY CO., LTD.) 22 August 2017 (2017-08-22) claims 1-10 | 1-9 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **05 December 2023** | **12 December 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/128149**

### C. DOCUMENTS CONSIDERED TO BE RELEVANT

| Category* | Citation of document, with indication, where appropriate, of the relevant passages | Relevant to claim No. |
|---|---|---|
| Y | CN 111701063 A (BEIJING XINGHANG ELECTRO-MECHANICAL EQUIPMENT CO., LTD.) 25 September 2020 (2020-09-25)<br>claims 1-10 | 2-9 |
| Y | CN 111397018 A (CIXI BEILIAN ELECTRICAL APPLIANCE CO., LTD.) 10 July 2020 (2020-07-10)<br>claims 1-10 | 2-9 |
| PX | CN 115721761 A (GUANGDONG HEJIE ELECTRICAL APPLIANCE CO., LTD.) 03 March 2023 (2023-03-03)<br>entire document | 1-9 |
| A | WO 2021240526 A1 (SHAARABANY EFFY) 02 December 2021 (2021-12-02)<br>entire document | 1-9 |
| A | US 2022373203 A1 (GUANGZHOU T.K. MEDICAL INSTRUMENT CO., LTD.) 24 November 2022 (2022-11-24)<br>entire document | 1-9 |

Form PCT/ISA/210 (second sheet) (July 2022)

## INTERNATIONAL SEARCH REPORT
### Information on patent family members

International application No.

**PCT/CN2023/128149**

| Patent document cited in search report | | | Publication date (day/month/year) | Patent family member(s) | | | Publication date (day/month/year) |
|---|---|---|---|---|---|---|---|
| CN | 111497571 | A | 07 August 2020 | None | | | |
| CN | 113350560 | A | 07 September 2021 | None | | | |
| CN | 107158908 | A | 15 September 2017 | None | | | |
| CN | 110721329 | A | 24 January 2020 | None | | | |
| CN | 206424767 | U | 22 August 2017 | None | | | |
| CN | 111701063 | A | 25 September 2020 | None | | | |
| CN | 111397018 | A | 10 July 2020 | None | | | |
| CN | 115721761 | A | 03 March 2023 | None | | | |
| WO | 2021240526 | A1 | 02 December 2021 | EP | 4158256 | A1 | 05 April 2023 |
| | | | | IL | 278580 | A | 01 December 2021 |
| | | | | IL | 278580 | B | 01 February 2022 |
| | | | | US | 2023241280 | A1 | 03 August 2023 |
| US | 2022373203 | A1 | 24 November 2022 | WO | 2022105031 | A1 | 27 May 2022 |
| | | | | EP | 4249007 | A1 | 27 September 2023 |

Form PCT/ISA/210 (patent family annex) (July 2022)